# EUROPEAN PATENT SPECIFICATION

(11) **EP 0 362 259 B1**
(45) Date of publication and mention of the grant of the patent: **06.03.1996**
(21) Application number: 88904935.9
(22) Date of filing: 20.05.1988
(51) Int. Cl.: C12P 21/02, C12N 15/00, C07K 14/00, A61K 38/16

(54) **METHOD OF PRODUCING CYSTATIN C OR MODIFICATIONS HEREOF AND DNA-SEQUENCE FOR USE WHEN CARRYING OUT THE METHOD**
VERFAHREN ZUR HERSTELLUNG VON CYSTATIN-C ODER ABWANDLUNGEN DAVON UND DNS-SEQUENZ ZUR AUSFÜHRUNG DIESER METHODE
PROCEDE DE PRODUCTION DE CYSTATINE C OU MODIFICATIONS DE LA CYSTATINE ET SEQUENCE D'ADN DESTINEE A ETRE UTILISEE LORS DE LA REALISATION DUDIT PROCEDE

(30) Priority: 22.05.1987 DK 2609/87
(43) Date of publication of application: 11.04.1990
(73) Proprietor: NOVO NORDISK A/S, DK-2820 Gentofte (DK)
(72) Inventor: GRUBB, Anders, S-222 47 Lund (SE); LUNDWALL, Ake, S-223 55 Lund (SE); ABRAHAMSON, Magnus, S-223 64 Lund (SE); DLABOGE, Henrik, DK-2830 Virum (DK)
(74) Representative: von Kreisler, Alek, Dipl.-Chem.
(86) International application number: DK8800082
(87) International publication number: WO8809384

(56) References cited:
- FEBS Letters, Volume 216, Number 2, 229-223, June 1987, (ABRAHAMSON et al) "Molecular Cloning and Sequence Analysis of cDNA Coding for the Precursor of the Human Cysteine Proteinase Inhibitor Cystatin"
- Proc. Natl. Acad. Sci. USA, Vol 83 pp 2974-2978, May 1986 (GHISO et al),
- Proc. Natl. Acad. Sci. USA, Vol 79. pp 3024-3027, May 1982, (GRUBB et al) "Human Y-Trace, a Basic Microprotein: Amino Acid Sequence and Presence in the Adenohypophysis"
- Biochemical and Biophysical Research Communications, Vol 86, No 3, 1979 (TONNELLE et al) "Partial Amino Acid Sequence of Two Forms of Human Post - Y - Globulin see Summary page 1, pp 613-619

## Description

The present invention relates to a method of producing cystatin C (previously called 8 -trace or post-8-globulin). The invention also relates to a DNA-sequence and a plasmid as well as a microorganism for use when carrying out the method.

Mature cystatin C is a known protein with 120 amino acids. The amino acid sequence is as follows:

Ser-Ser-Prq-Gly-Lys-Pro-Pro-Arg-Leu-Val-Gly-Gly-Pro-Met-
Asp-Ala-Ser-Val-Glu-Glu-Glu-Gly-Val-Arg-Arg-Ala-Leu-Asp-
Phe-Ala-Val-Gly-Glu-Tyr-Asn-Lys-Ala-Ser-Asn-Asp-Met-Tyr-
Mis-Ser-Arg-Ala-Leu-Gln-Val-Val-Arg-Ala-Arg-Lys-Gln-Ile-
Val-Ala-Gly-Val-Asn-Tyr-Phe-Leu-Asp-Val-Glu-Leu-Gly-Arg-
Thr-Thr-Cys-Thr-Lys-Thr-Gln-Pro-Asn-Leu-Asp-Asn-Cys-Pro-
Phe-His-Asp-Gln-Pro-Mis-Leu-Lys-Arg-Lys-Ala-Phe-Cys-Ser-
Phe-Gln-Ile-Tyr-Ala-Val-Pro-Trp-Gln-Gly-Thr-Met-Thr-Leu-
Ser-Lys-Ser-Thr-Cys-Gln-Asp-Ala

Native human cystatin C is a mixture of two types differing in that type I has the amino acid sequence Ser-Ser-OHPro-Gly...Ala, while type II, called 3-des-OH-cystatin C, has the following sequence: Ser-Ser-Pro-Gly...Ala (1). Thus, the two types only differ in the third amino acid residue. As the relative ratio between the two types may vary somewhat, dependent on the origin of the preparations and the extraction method, the biological activity of the preparations may also vary somewhat.

Cystatin C prepared according to the invention is of the type 3-des-OH-cystatin C and thus is considered to have constant activity.

As is known from the literature, cystatin C is an efficient inhibitor for cystein-proteinases, i.a. papain and cathepsin B.

Cystein-proteinases participate in the intracellular catabolism of proteins and peptides, in the proteolytical conversion of prohormones, in the extracellular degradation of collagen and in the penetration of normal tissue with malignant cells.

As cystatin C inhibts cystein-proteinases which accelerate cancer growth or metastasis-formation, it is a potential cancer-inhibiting agent.

Furthermore, cystatin C possesses antiviral and possibly antibacterial activity.

Cystein-proteinases, e.g. chymopapain, may be used for treatment of slipped disc. If these cystein-proteinases diffuse into the cerebrospinal fluid, cerebral hemorrhage will occur as a serious side-effect of the therapeutic treatment of slipped disc. As cystatin C inhibits such side-effect, it has therapeutic use in this connection.

Particularly important is the activity of cystatin C to counteract hereditary inclination to cerebral hemorrhage with amyloidosis. It is deposited as amyloid in the arterial walls. The metabolism of cystatin C in this decease which attacks young people is changed, and the sequence for deposited cystatin C deviates at one position from what originally is described regarding cystatin C isolated from people without the decease.

In certain types of hereditary cerebral hemorrhage cystatin C is precipitated after splitting off the 10 amino-terminal amino acids in the form of an amyloid in the cerebral arteries which burst. In case of these types of hereditary cerebral hemorrhage a cystatin C molecule is present which at position 68 has a glutamine acid residue instead of a leucine residue.

In the decomposition of collagen in tissue participate i.a. cystein-proteinases, and cystatin C may therefore probably inhibit such decomposition of tissue.

The cystatin C amino acid sequence isolated from human urine has been characterized and appears to be somewhat homologous to c-Ha-ras oncogene products.

The invention is based on the use of a novel DNA-sequence containing codons which code for cystatin C or modifications thereof.

One embodiment of the invention relates to a DNA-copy or a cDNA-sequence formed on the basis of human cells or placenta. Thus, the invention also relates to such DNA-sequence, the structure of which is stated in claim 2.

Further, the invention relates to modified cystatin C, in which one or more amino acids at positions 5-17, 55-59 and/or 68 have been replaced by another amino acid. An example of such modified cystatin C is a derivative in which Leu at position 68 is changed to Gln.

The invention also relates to a method of producing biosynthetic cystatin C or a modified cystatin C. In such a method a host organism is used, such as bacterium, a yeast cell or a mammal cell line, in which a vector is included, such as a plasmid having the structure suited for expressing cystatin C or a modified cystatin C.

The DNA-sequence in question is inserted together with signal sequences, promotors etc in a plasmid incorporated in the microorganism which is subsequently cultured in a manner known per se.

In the method according to the invention a host organism containing a DNA-sequence coding for cystatin C is cultured in a substrate, the cystatin C formed subsequently being extracted from the culture medium. The preferred host organism is E. coli, but in principle any microorganism may be used, including yeast cells such as Saccharomyces Cerevisiae, or culturing can proceed by means of mammalian cells.

The cystatin C formed is extracted from the culture medium in a manner known per se, e.g. by extraction by way of solvents and purification by chromatography. Affinity chromatography with monoclonal or polyclonal anti-body substances has turned out particularly suited for isolating cystatin C in pure state.

Isolation and analysis of cDNA clones have been effected by screening recombinant phages. Thus λ gtll cDNA library from human placenta and liver mRNA have been used. Screening was at a density of 50.000 plaques pr. 130 mm petri-dishes with affinity-purified anti-body substance by a method according to Young and Davis. Bound anti-body substance was demonstrated by alkaline phosphatase-conjugated anti-substance (Promega Bioteknik).

λ-phages produced by the plate lysat method were isolated by centrifugation in a CsCl gradient. DNA was extracted by standard methods. A mixed olignonucleotide probe being specific for cystatin C was hybridized to the product of phage DNA reacted with EcoRI in a Southern blot experiment. Hybridized cDNA insertion was ligated in EcoRI-linearized pUC18 plasmid vectors which were then used for transforming E. coli JMB83 cells. Plasmids were produced by alkaline lysis method. DNA-sequencing of insertions, the sub-clone in M13mp8, was carried out using a modified dideoxy-chain terminator technique.

In the following the invention is described by way of examples.

### Example 1

cDNA library from human cells from liver, prostata and placenta were screened for cystatin C coding clones. About 1.2 x10⁶ recombinants of liver-cells and 3 x 10⁵ recombinants from prostata cells were screened with anti-substances and a mixed oligonucleotide probe. The result of this screening was negative. On the other hand screening of 6 x 10⁵ recombinants of placenta cells yielded nine clones which reacted with the anti-substance.

Three of these clones, designated C5, C6a and C12 respectively, were used in the following. Their inserts of 800, 800 and 700 base pairs, respectively, were specifically hybridized by Southern blot experiments to a mixed ologonucleotide, constructed from protein-sequence-data.

By sequencing inserts from clones C6a and C12, the sub-clone in EcoRI-cut M13mp8, it was disclosed that the inserts shared the same 3'-sequence, containing the polyadenyl-ring signal AATAAA.

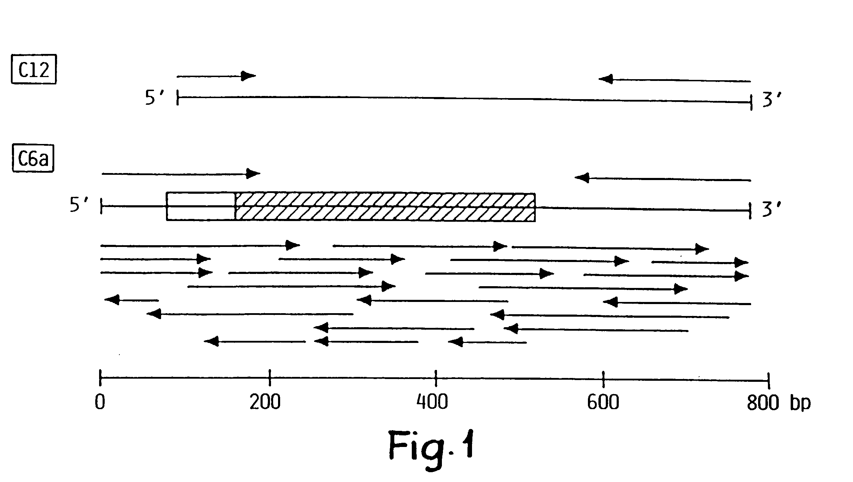

The entire sequence of both strings of the clone C6a cDNA insert was determined by sequencing randonmnly overlapping fragments, the sub-clone in M13mp8. Each nucleotide was determined on an average 5.63 times. Data achieved by sequencing cDNA inserts of clones C6a and C12 from the ends revealed that the sequence of C12 inserts starts at position 91 of the C6a, see fig. 1.

The C6a insert contains 777 base pairs, including 77 base pairs of 5'-non-coding sequence and 262 base pairs of 3'-non-coding sequence. The poladenyl signal AATAAA at position 756-761 is succeeded by 15 nucleotides. A possible translation initiation in agreement with the sequence discovered by Kozak is found 6 bp downstream of a stop codon. Initiation at this position and translation stop at the TAG-code at positions 516-518 will finally result in a 120 amino acid long cystatine C with a signal sequence of 26 amino acid residues. The protein in question will be identical with cystatin C, isolated from urine.

Fig. 1 below illustrates a sequencing strategy for human cystatin C cDNA-inserts. Inserts of the clones C12 and C6a were sequenced from their ends by the chain-terminator-method. A "shotgun" sequencing-analysis technique was used for sequencing both DNA-strings of C6a inserts. The horizontal arrows indicate the direction and extension of each sequencing analysis. The protein-coding area for inserted C6a is boxed and the hatched part indicates the area which codes for mature protein.

The DNA-structure coding for cystetin C comprises nucleotides derived from amino acid sequence for the clone C6a cDNA which codes for human precystatin C. The numbering of the nucleotid sequence starts at the first nucleotide and proceeds in the direction from 5' to 3'. The numbering of the amino acid starts with the first amino acid in the mature protein. The amino acid substituted in the cystatin C fragment deposited as amyloid in patients suffering from hereditary cerebral hemorrhage with amyloidosis is boxed. The Kozak-initiation and the polyadenyl-ring signal are underlined.

### Example 2

### Cytoplasmatic expression of methionin extended Cystatin C in E.coli:

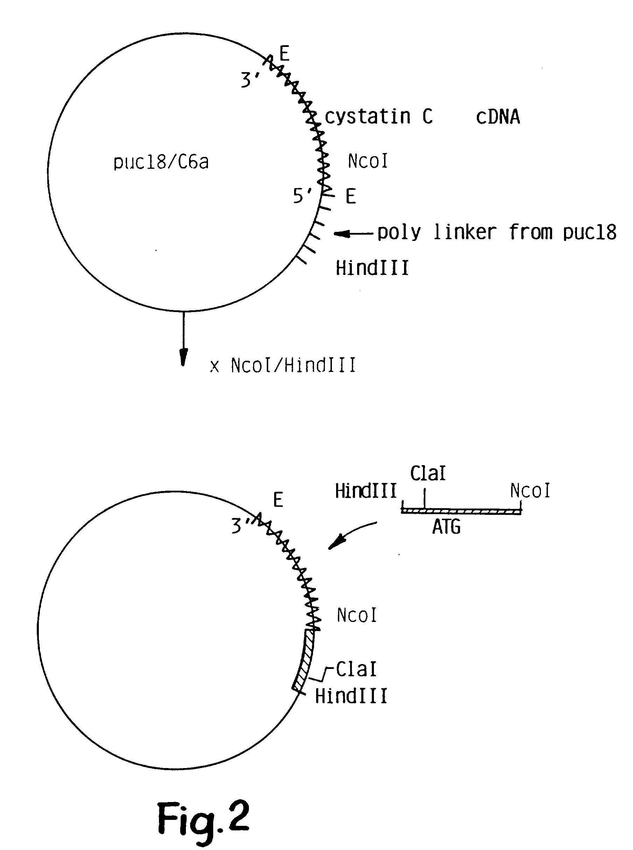

The plasmid pUC18/C6a containing the cDNA sequence for Cystation C was cut by the restriction enzymes NcoI/HindIII, whereafter the plasmid fragment was purified. A synthetic DNA linker containing the code sequence for the amino acid methionin and the approx. first 40 bases of mature Cystatin C were subsequently introduced between the NcoI and the HindIII sites (fig. 2). Instead of the codons occurring in the cDNA of cystatin C codons were used in the linker which result in high protein yield in E.coli. This plasmid was cut by the restriction enzymes ClaI/EcoRI, whereafter a fragment of about 600 by containing the code sequence for methionin-extended Cystatin C was purified.

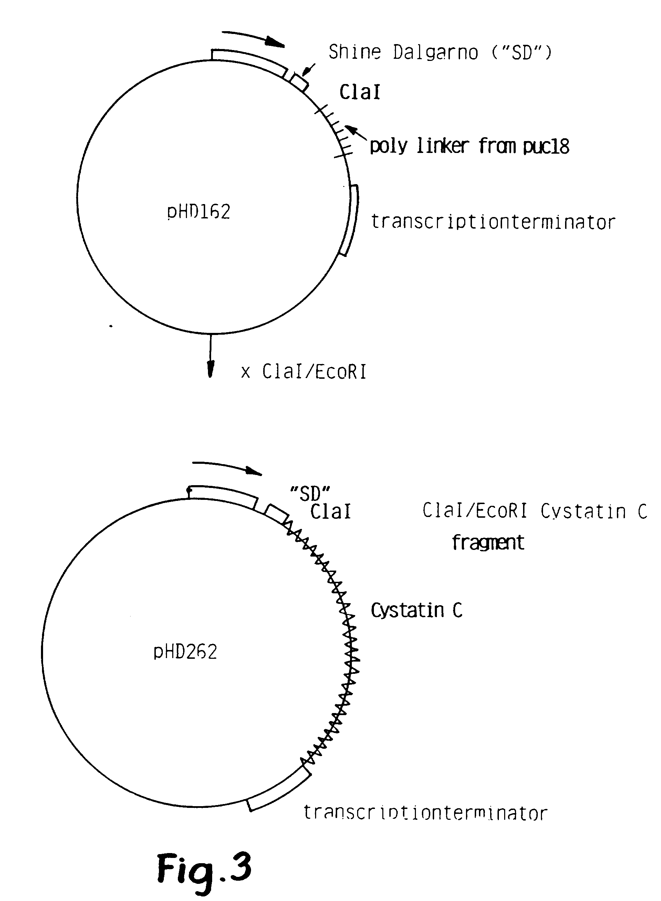

The plasmid pHD162 containing a synthetic promotor and Shine and Dalgarno sequence was cut by the restriction enzymes ClaI/EcoRI, whereafter the plasmid fragment was purified. This plasmid fragment was subsequently combined with the above 600 bp fragment to form the Cystatin C expression plasmid pHD262 (coding for methionin Cystatin C) (fig. 3). E.coli MC1061 containing the above expression plasmid was cultured as described below.

### Example 3

### Periplasmatic expression of Cystatin C in E.coli:

The plasmid pUC18/C6a mentioned in example 2 was cut by the restriction enzymes NcoI/HindIII, whereafter the plasmid fragment was purified. A synthetic DNA linker containing the code sequence for Outer membrane protein A (OmpA) from E.coli and the first approx. 40 bases of mature Cystatin C were subsequently inserted between the NcoI and HindIII sites. Optimal E.coli codons were used. This plasmid was subsequently cut by the restriction enzymes ClaI/EcoRI, whereafter a fragment of about 700 bp containing the code sequence for the OmpA signal peptide and Cystatin C were purified.

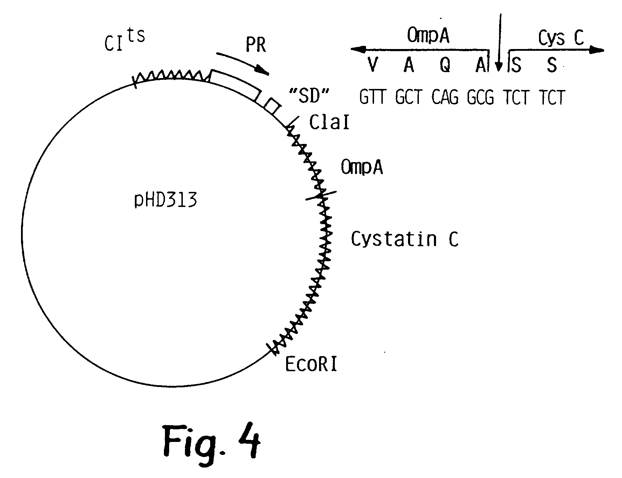

The above fragment was then introduced in an expression plasmid containing the λPR promotor, an optimal Shine and Dalgarno sequence, the polylinker region from the plasmide pUC18 and the temperature sensitive CI repressor gene from λ. The plasmid pHD313 (fig. 4) hereby formed was then introduced in E.coli MC1061. The expression of Cystatin C and purification of Cystatin C were carried out as herein described.

### Example 4

### Periplasmatic expression of cystatin C in E.coli:

The plasmid pUC18 was cut by ApaI/EcoRI, and a fragment of approx. 680 base pairs was purified and ligated onto a synthetic ApaI/ClaI linker containing the code sequence for about the first 3 amino acids of precystatin C. The fragment was subsequently introduced into the expression plasmid, mentioned in example 3, containing the temperature inducing λPR promotor.

### Example 5

### Expression of periplasmatic modified Cystatin C in E.coli:

A HindIII/EcoRI fragment of about 700 bp was isolated from the expression plasmid pHD313 (example 3).The fragment was introduced in M13 MP18 and exposed to in vitro mutagenesis so that the code sequence for Cystatin C was changed to contain one or more of the modifications appearing from table 1 below. After in vitro mutagenesis a number of clones were cultured and sequence determined. Clones with correct sequence were subsequently cut by ClaI/EcoRI, whereafter a fragment of about 700 bp was isolated and introduced into the expression plasmid pHD313. The plasmides hereby formed were cultured, and the product purified as described above.

### Table 1

### CYSTATIN C MUTANTS WITH MODIFIED DNA-SEQUENCE

1. Leu⁶⁸→Gln
2. Del.Ser¹-Val¹⁰
3. 1+2
4. Gly¹¹→pos. charged, e.g. Arg
5. Gly¹¹→neg. charged, e.g. Glu
6. Gly¹¹→strongly hydrofob., e.g. Trp
7. 4.Gly¹¹→Ala¹¹, 5Gly¹¹→Ser¹¹
8. Substitution in single amino acids, e.g.
   a.) Arg⁸→Glu
   b.) Leu⁹→Arg,Glu
   c.) Val¹⁰→Arg,Glu
   d.) Gly¹→Arg,Glu
   e.) Pro¹³→Arg,Glu,Trp
   f.) Arg¹⁴→Arg,Glu
9. Substitution of the sub-regions, e.g. Arg⁸-Met¹⁴→ Gly-Gly-Gly-Gly-Gly-Gly-Gly
10. Deletion Arg⁸-Met¹⁴
11. Cys⁸³→Gly
12. Cys⁹⁷→Gly
13. Deletion Asn⁶¹→Ala¹⁰
14. Deletion Asp⁸¹→Ala¹⁰
15. Deletion Ile¹⁰¹→Ala¹⁰

### Example 6

Production of Cystatin C on a technical scale by batch fermentation.

E.coli MC1061 pHD 313-1-1, which expresses Cystatin C with correct N-terminal, is cultured on LB petri dish containing ampicilline. A single colony is transferred to a 100 ml shaker flask containing LB medium and 50 mg/l ampicilline. This is incubated at 30°C, 200 rpm for 20 hours.

The fermentation is carried out in 10 1 laboratory fermentor under the following conditions: pH = 7.2, aeration = 1 VVM, stirring = 1000 rpm and temperature = 30°C in the first phase. The medium used consists of yeast extract, casamino acids and various salts.

The fermentor is inoculated with 100 ml culture from the shake flask and the dosing of glucose starts when the biomass contentration is OD₅₂₅ₙₘ = 5. The feed rate must be 3.5 g glucose/l.h, and it is maintained throughout the process. The formation of Cystatin C is induced by raising the temperature in the fermentor to 40°C in the growth phase (OD₅₂₅ₙₘ = 50) Cystatin C is formed quickly - 2 hours after the temperature was raised the fermentation is ended by lowering the temperature to 20°C and raising pH to 9.0. The entire fermentation process lasts about 12 hours.

By the process are achieved final concentrations of Cystatin C of approx. 1000 mg/l (Cystatin C constitutes approx. 10% of the total protein in the cell).

### Extraction of Cystatin C

### Extraction I of Cystatin C from E.coli

The cell suspension from the fermentation is centrifuged. The cells are resuspended in 20-25% w/w sucrose, 0.1 M EDTA, 0.2M Tris pH 9.0 and are agitated for 20 min. Cells are removed by centrifugation and the pellet is quickly resuspended in 0°C 10mM Tris pH 9 under vigorous agitation for 10 min.

The supernatant herefrom contains Cystatin C from the periplasmatic phase (70% of total extraction by mechanical homogenization).

### Extraction II of Cystatin C from E.coli

Cell suspension from fermentation is adjusted to pH = 10.5 with 5M NaOH. After stirring for 20 min. the suspension is centrifuged. The supernatant herefrom contains Cystatin C.

### Extraction III of Cystatin C from E.coli

Cell suspension from fermentation is centrifuged and resuspended in 0.5M Tris pH = 10.5. After stirring for 20 min. the suspension is centrifuged. The supernatant herefrom contains Cystatin C.

### Example 7

The extract is dialyzed against 20 nM ethanolamine pH = 10.0 with a dialyze membrane of the type Spectropor^{®} (mw cut 3500) and is then subjected to a Q-Sepharose column equilibrated in 20 mM ethanolamine, pH = 10.0. Fractions containing Cystatin C (3-des-OH cystatin C) are combined and concentrated on a filter of the type Diaflo^{®}YM2. Then the material is subjected to a column of the type Bio-Gel^{®}P60, equilibrated in 0.05 M ammonium hydrogen carbonate. Fractions containing Cystatin C are collected. By this isolation procedure the yield is typically 50-60%

### Construction of a synthetic gene coding for Cystatin C by using optimal E.coli codons:

It is known from the literature that highly expressed E.coli proteins make the use of certain condons for the individual amino acids. Simultaneously with the production of cDNA for Cystatin C a Cystatin C gene was synthesized on the basis of the published amino acid sequence, the gene contained the codons preferred in highly expressed E.coli proteins. The gene was also modified in such a way as to allow a fusion with the signal sequence from either outer membrane protein A from E.coli or the signal sequence from the fibriaprotein K88/99 from E.coli. After synthesis the above gene was step-cloned in the plasmid pUC18. The gene was hereafter isolated and introduced in the expression plasmid mentioned in example 3 in combination with one of the above signal sequences. The plasmid was introduced into E.coli MC1061 and cultured as described above.

OmpA-cystatin C gene with optimal E.coli codons.

CG-ATG-AAA-AAA-ACT-GCT-ATC-GCT-ATC-GCT-GTT-GCT-CTG-GCT-
GGT-TTC-GCT-ACT-GTT-GCT-CAG-GCT-TCT-TCT-CCT-GGT-AAA-CCG-
CCT-CGT-CTG-GTT-GGT-GGT-CCG-ATG-GAC-GCT-TCT-GTT-GAA-GAA-
GAA-GGT-GTT-CGT-CGT-GCT-CTG-GAC-TTC-GCT-GTT-GGT-GAA-TAC-
AAC-AAA-GCT-GTT-AAC-GAC-ATG-TAC-CAC-TCT-CGT-GCT-CTG-CAG-
GTT-GTT-CGT-GCT-CGT-AAA-CAG-ATC-GTT-GCT-GGT-GTT-AAC-TAC-
TTC-CTG-GAC-GTT-GAA-CTG-GGT-CGT-ACC-ACC-TGC-ACC-AAA-ACC-
CAG-CCG-AAC-CTG-GAC-AAC-TGC-CCG-TTC-CAC-GAC-CAG-CCG-CAC-
CTG-AAA-CGT-AAA-GCT-TTC-TGC-TCT-TTC-CAG-ATC-TAC-GCT-GTT-
CCG-TGG-CAG-GGT-ACC-ATG-ACC-CTG-TCT-AAA-TCT-ACC-TGC-CAG-
GAC-GCT-TAA-TAG

### Example 8

### Production and isolation of 3-des-OH-cystatin C

Bacteria media containing 3-des-OH-cystatin C were centrifuged and bacteria-free media concentrated on a Diaflo^{™} YM 2 filter. The concentrated material was dialyzed against 20 mM etanol amine, pH 10.0 in Spectrapor^{™} dialysis membranes (mw cutoff 3.500) and subsequently applied to a O-Sepharose^{™} column equilibrated in 20 mM etanol amine, pH 10.0. Fractions containing 3-des-OH-cystatin C were pooled and concentrated on a Diaflo^{™} YM 2 filter. The material was then applied to a Bio-Gel™ column equilibrated in 0.05 M ammonium bicarbonate. Fractions containing 3-des-OH-cystatin C were combined. By this isolation procedure the 3-des-OH-cystatin C yield is typically 50-60%.

### Characterization of 3-des-OH-cystatin C:

3-des-OH-cystatin C isolated as described above from culture of pHD 313-transformed E.coli, was physically-chemically characterized with a view to biological activity. The results from the characterization were compared with result from corresponding analysis of human cystatin C.

Analysis with Ouchterlony's double immune diffusion technique showed that all cystatin C-epitopes recognized by a polyclonal anti-serum raised against human cystatin C are also present in 3-des-OH-cystatin C. Agarose gel electrofores at pH 8.6 and SDS-PAGE under reducing or non-reducing conditions showed that 3-des-OH-cystatin C and human cystatin C are identical as regards charge and molecular weight. Amino acid analysis showed that the amino acid composition in 3-des-OH-cystatin C is almost identical with that of human cystatin C. Automated sequence determination showed that 3-des-OH-cystatin C has the N-terminal sequence.

SSPGKPPRLVGGPMDASVEEEGV--ALDFAVGEYNKASNDMY at each position identical with that found in human cystatin C (cf. ref. 1) and in addition 100% of the prolin residue at position 3 in 3-des-OH-cystatin C lacked a hydroxyl group. Amino acid analysis of disulfide bound peptides showed that 3-des-OH-cystatin C has disulfide bridges between Cys⁷³-Cys⁸³ and Cys⁷³-Cys⁸³, i.e. the same as is the case in human cystatin C (cf. ref. 15).

Biological activity in 3-des-OH-cystatin C is determined through titration against concentration determined papain. 3-des-OH-cystatin C was 83% active, which is higher than corresponding figures for human cystatin C (55%). The strength of binding 3-des-OH-cystatin C on the cystein proteinases papain (EC 3.4.22.2), cathepsin B (EC 3.4.22.1) and dipeptidyl peptidase I (EC 3.4.14.1) was determined. The equilibrium constant for dissociation of 3-des-OH-cystatin C enzyme complex (< 0.005 nM, 0.50 nM and 3.5 nM) was within the experimental error limit identical with the equilibrium constant for human cystatin C enzyme complex (< 0.005 nM, 0.27 nM and 3.5 nM).

Thus physical-chemical characterization and analysis performed of the interaction of 3-des-OH-cystatin with cystein proteinases shows that 3-des-OH-cystatin C has full biological activity and is identical with human cystatin C with the exception that it completely lacks hydroxyl group on residue 3.

### Example 9

### Therapeutic use of 3-des-OH-cystatin C as a CNS-protective agent at chymopapin-treatment of sciatica

Just before chymopapin treatment of patients with sciatica a molar amount of 3-des-OH-cystatin C three times higher than the molar amount of chymopapin to be used is injected into the cerebrospinal fluid. For example, if 8 mg of chymopapin (the active component of the currently available drugs: Discase®; Travenol Laboraties Ltd., Thetford, UK and Chymodiactin®; Smith Laboraties, Inc., Rosemont, IL, U.S.A.) is to be used for intradiscal injection, 12 mg of 3-des-OH-cystatin C is injected into the cerebrospinal fluid. The 3-des-OH-cystatin C might be composed of 5 mg 3-des-OH-cystatin C per ml of sterile physiological saline. This will protect against any side effects due to proteolytic breakdown of nervous tissue caused by improper injection of chymopapain into the cerebrospinal fluid.

Alternatively, if clinical or radiographic signs of improper chymopapain injection into the cerebrospinal fluid are observed at, or after, execution of the intended intradiscal chymopapain injection, a molar amount of 3-des-OH-cystatin C three times higher than the molar amount of chymopapain used, is immediately injected into the cerebrospinal fluid in order to inhibit the tissue-damaging effect of chymopapain.

Directly after the injection of 3-des-OH-cystatin C the patient is gently moved in a way which brings about a rapid distribution of the injected solution of 3-des-OH-cystatin C with the cerebrospinal fluid of the patient.

### Literature

1. Grubb, A & Löfberg, Lt: Proe. Natl. Acad. Sci. 79, 3024-3027, 1982
2. Abrahamson M, Barrett A J, Salvesen G, Grubb A, J. Biol Chem 1986;261(24):1182-9.
3. Abrahamson M, Ritonja A, Brown M A, Grubb A, Machleidt W, Barrett A, J. Biol Chem 1987;262(20):9688-94.
4. Barrett A J, Biochem Sci 1987;12(5):193-6.
5. Barrett A J, Fritz H, Grubb A, Isemura S, Jarvinen M, Katunuma N, Machleidt W, Muller Esterl W, Sasaki M, Turk V, Biochem J 1986;236(1):312.
6. Buttle D J, Abrahamson M, Barrett A J, Spine 1986;11(7):688-94.
7. Delaissé J M, Eeckhout Y, Vaes G, Biochem Biophys Res. Commun 1984;125(2):441-7.
8. Ford L T, Clin Orthop 1969;67:68-71.
9. Garvin P J, Jennings R B, Smith L, Gesler R M, Chymopapain, Clin Orthop 1965;41:204-23.
10. Grubb A, Jensson O, Gudmundsson G, Arnason A, Löfberg H, Malm J, N Engl J Med 1984;311(24);1547-9.
11. Grubb A, Grimsberg V, Grubb A, Acta Neurol Scand 1986;73:309-310.
12. Jensson O, Gudmundsson G, Arnason A, Blöndal H, Petrusdottir I, Thorsteinsson L, Grubb A, Löfberg H, Cohen D, Frangione B, Acta Neurol Scand 1987;76:102-14.
13. Nachemson A L, Rydevik B, Acta Orthop Scand 1988;59(1):56-62.
14. Grubb A; Acta Orthop Scand 1988;59(1):63-65.
15. Grubb A; Löfberg H & Barrett A.,J FEBS Lett. 170.370-374,1984

## Claims

1. Polypeptide having increased cystatin C activity, **characterized** in that it consists of, by recombinant technique produced, 3-des-hydroxy-cystatin C or a modification thereof, wherein one or more amino acids in the positions 5-17 and/or 55-59, 83 and 97 have been replaced by other amino acids, and/or wherein one or more sequences in positions 8-14 and/or 61-120 have been deleted.

2. DNA-sequence for the expression of a polypeptide according to claim 1, **characterized** in that it is a DNA-copy or CDNA having the structure 
22
GGGCGCAGCGGTCCTCTCTAT

95
CTAGCTCCAGCCTCTCGCCTGCGCCCCACTCCCCGCGTCCCGCTCCTAGCCGACC ATG GCC GGG CCC CTG CGC
Met Ala Gly Pro Leu Arg
-26 -21

155
GCC CCG CTG CTC CTG CTG GCC ATC CTG GCC GTG GCC CTG GCC GTG AGC CCC GCG GCC GGC
Ala Pro Leu Leu Leu Leu Ala Ile Leu Ala Val Ala Leu Ala Val Ser Pro Ala Ala Gly
-1

215
TCC AGT CCC GGC AAG CCG CCG CGC CTG GTG GGA GGC CCC ATG GAC GCC AGC GTG GAG GAG
Ser Ser Pro Gly Lys Pro Pro Arg Leu Val Gly Gly Pro Met Asp Ala Ser Val Glu Glu
1 20

275
GAG GGT GTG CGG CGT GCA CTG GAC TTT GCC GTC GGC GAG TAC AAC AAA GCC AGC AAC GAC
Glu Gly Val Arg Arg Ala Leu Asp Phe Ala Val Gly Glu Tyr Asn Lys Ala Ser Asn Asp
40

335
ATG TAC CAC AGC CGC GCG CTG CAG GTG GTG CGC GCC CGC AAG CAG ATC GTA GCT GGG GTG

60

395
AAC TAC TTC TTG GAC GTG GAG CTG GGC CGA ACC ACG TGT ACC AAG ACC CAG CCC AAC TTG

80

455
GAC AAC TGC CCC TTC CAT GAC CAG CCA CAT CTG AAA AGG AAA GCA TTC TGC TCT TTC CAG

100
 
515
ATC TAC GCT GTG CCT TGG CAG GGC ACA ATG ACC TTG TCG AAA TCC ACC TGT CAG GAC GCC

120

593
TAG GGGTCTGTACCGGGCTGGCCTGTGCCTATCACCTCTTATGCACACCTCCCACCCCCTCTATTCCCACCCCTGGAC

672
TGGTGGCCCCTGCCTTGGGGAAGGTCTCCCCATGTGCCTGCACCAGGAGACAGACAGAGAAGGCAGCAGGCGGCCTTTG

751
TTGCTCAGCAAGGGGCTCTGCCCTCCCTCCTTCCTTCTTGCTTCTCATAGCCCCGGTGTGCGGTGCATACACCCCCACC

777
TCCTGCAATAAAATAGTAGCATCCCC
 or a fraction of this structure

3. DNA-sequence, **characterized** in that it comprises codons coding for a modified 3-des-hydroxy-cystatin C as stated in claim 1.

4. DNA-sequence, **characterized** in that it comprises codons coding for 3-des-hydroxy-cystatin C with one or more of the following modifications:
1. Gly¹¹ pos. charged, e.g. Arg
2. Gly¹¹→ neg. charged, e.g. Glu
3. Gly¹¹→ strongly hydrofob., e.g. Trp
4. 4.Gly¹¹-Ala¹¹, 5Gly¹¹-Ser¹¹
5. Substitution in single amino acids, e.g.
a) Arg⁸-Glu
b) Leu⁹-Arg,Glu
c) Val¹⁰→Arg,Glu
d) Gly¹→Arg,Glu
e) Pro¹³→Arg,Glu,Trp
f) Art¹⁴→Arg,Glu
6. Substitution of the entire region, e.g. Arg⁸→met¹⁴→ Gly-Gly-Gly-Gly-Gly-Gly-Gly
7. Deletion Arg⁶-Met¹⁴
8. Cys⁸³→Gly
9. Cys⁹⁷→Gly
10. Deletion Asn⁶¹→Ala¹⁰
11. Deletion Asp⁸¹→Ala¹⁰
12. Deletion Ile¹⁰¹-Ala¹⁰

5. A method of producing 3-des-hydroxy-cystatin C or a modification thereof according to claim 1, **characterized** in that a host organism containing a DNA-sequence as specified in claim 2, 3 or 4 is cultured in a substrate, the 3-des-hydroxy-cystatin C or the modification thereof formed subsequently being extracted from the culture.

6. Plasmid for introduction into a host organism which is used for biosynthetic fermentation of 3-des-hydroxy-cystatin C or a modification thereof according to claim 1, **characterized** in that it contains the DNA-structure stated in claims 2, 3 or 4 as well as promotor and signal sequences etc.

7. Microorganism, **characterized** in that it contains a plasmid as stated in claim 6.

8. Therapeutic preparation, **characterized** in that it contains 3-des-hydroxy-cystatin C or a modification thereof as defined in claim 1.

## Patentansprüche

1. Polypeptid mit erhöhter Cystatin-C-Aktivität, dadurch gekennzeichnet, daß es aus durch Rekombinationstechnik erzeugtem 3-Deshydroxycystatin C oder einer Modifikation davon, bei der eine oder mehrere Aminosäuren in den Positionen 5-17 und/oder 55-59, 83 und 97 durch andere Aminosäuren ersetzt wurden und/oder bei der eine oder mehrere Sequenzen in den Positionen 8-14 und/oder 61-120 entfernt wurde, besteht.

2. DNA-Sequenz für die Expression eines Polypeptids nach Anspruch 1, dadurch gekennzeichnet, daß es sich um eine DNA-Kopie oder cDNA mit der Struktur 
22
GGCCGCAGCGGTCCTCTCTAT

95
CTAGCTCCAGCCTCTCGCCTGCGCCCCACTCCCCGCGTCCCGCTCCTAGCCGACC ATG GCC GGG CCC CTG CGC
Met Ala Gly Pro Leu Arg
-26 -21

155
GCC CCG CTG CTC CTG CTG GCC ATC CTG GCC GTG GCC CTG GCC GTG AGC CCC GCG GCC GGC
Ala Pro Leu Leu Leu Leu Ala Ile Leu Ala Val Ala Leu Ala Val Ser Pro Ala Ala Gly
-1

215
TCC AGT CCC GGC AAG CCG CCG CGC CTG GTG GGA GGC CCC ATG GAC GCC AGC GTG GAG GAG
Ser Ser Pro Gly Lys Pro Pro Arg Leu Val Gly Gly Pro Met Asp Ala Ser Val Glu Glu
1 20

275
GAG GGT GTG CGG CGT GCA CTG GAC TTT GCC GTC GGC GAG TAC AAC AAA GCC AGC AAC GAC
Glu Gly Val Arg Arg Ala Leu Asp Phe Ala Val Gly Glu Tyr Asn Lys Ala Ser Asn Asp
40

335
ATG TAC CAC AGC CGC GCG CTG CAG GTG GTG CGC GCC CGC AAG CAG ATC GTA GCT GGG GTG

60

395
AAC TAC TTC TTG GAC GTG GAG CTG GGC CGA ACC ACG TGT ACC AAG ACC CAG CCC AAC TTG

80

455
GAC AAC TGC CCC TTC CAT GAC CAG CCA CAT CTG AAA AGG AAA GCA TTC TGC TCT TTC CAG

100

515
ATC TAC GCT GTG CCT TGG CAG GGC ACA ATG ACC TTG TCG AAA TCC ACC TGT CAG GAC GCC

120

593
TAG GGGTCTGTACCGGGCTGGCCTGTGCCTATCACCTCTTATGCACACCTCCCACCCCCTCTATTCCCACCCCTGGAC

672
TGGTGGCCCCTGCCTTGGGGAAGGTCTCCCCATGTGCCTGCACCAGGAGACAGACAGAGAAGGCAGCAGGCGGCCTTTG

751
TTGCTCAGCAAGGGGCTCTGCCCTCCCTCCTTCCTTCTTGCTTCTCATAGCCCCGGTGTGCGGTGCATACACCCCCACC

777
TCCTGCAATAAAATAGTAGCATCCCC
 oder einem Bruchstück dieser Struktur handelt.

3. DNA-Sequenz, dadurch gekennzeichnet, daß sie Codons umfaßt, die für ein modifiziertes 3-Deshydroxycystatin C gemäß Anspruch 1 codieren.

4. DNA-Sequenz, dadurch gekennzeichnet, daß sie Codons umfaßt, die für 3-Deshydroxycystatin C mit einer oder mehreren der folgenden Modifikationen codieren:
1. Gly¹¹ → pos. geladen, z.B. Arg
2. Gly¹¹ → neg. geladen, z.B. Glu
3. Gly¹¹ → stark hydrophob, z.B. Trp
4. 4.Gly¹¹ → Ala¹¹, 5Gly¹¹ → Ser¹¹
5. Substitution bei einzelnen Aminosäuren, z.B.
a) Arg⁸ → Glu
b) Leu⁹ → Arg,Glu
c) Val¹⁰ → Arg,Glu
d) Gly¹ → Arg,Glu
e) Pro¹³ → Arg,Glu,Trp
f) Met¹⁴ → Arg,Glu
6. Substitution des gesamten Bereichs, z.B. Arg⁸→Met¹⁴→ Gly-Gly-Gly-Gly-Gly-Gly-Gly
7 . Deletion Arg⁸→Met¹⁴
8. Cys⁸³ → Gly
9. Cys⁹³ → Gly
10. Deletion Asn⁶¹→Ala¹⁰
11. Deletion Asp⁸¹→Ala¹⁰
12. Deletion Ile¹⁰¹→Ala¹⁰

5. Verfahren zur Herstellung von 3-Deshydroxycystatin C oder einer Modifikation davon gemäß Anspruch 1, dadurch gekennzeichnet, daß ein Wirtsorganismus, der eine DNA-Sequenz gemäß Anspruch 2, 3 oder 4 enthält, in einem Substrat kultiviert wird, wobei das gebildete 3-Deshydroxycystatin C oder die Modifikation davon anschließend aus der Kultur extrahiert wird.

6. Plasmid zur Einführung in einen Wirtsorganismus, der zur biosynthetischen Fermentation von 3-Deshydroxycystatin C oder einer Modifikation davon gemäß Anspruch 1 verwendet wird, dadurch gekennzeichnet, daß es die DNA-Struktur gemäß Anspruch 2, 3 oder 4 sowie Promotor- und Signalsequenzen usw. enthält.

7. Mikroorganismus, dadurch gekennzeichnet, daß er ein Plasmid gemäß Anspruch 6 enthält.

8. Therapeutisches Präparat, dadurch gekennzeichnet, daß es 3-Deshydroxycystatin C oder eine Modifikation davon gemäß Anspruch 1 enthält.

## Revendications

1. Polypeptide ayant une activité renforcée de cystatine C, caractérisé en ce qu'il consiste en une substance produite par génie génétique qui est la 3-déshydroxycystatine C ou un de ses dérivés dans lesquels un ou plusieurs aminoacides en position 5-17 et/ou 55-59, 83 et 97 ont été remplacés par d'autres aminoacides, et/ou dans lesquels une ou plusieurs séquences en position 8-14 et/ou 61-120 ont été supprimées.

2. Séquence d'ADN pour l'expression d'un polypeptide selon la revendication 1, caractérisée en ce qu'il s'agit d'une copie d'ADN ou d'un ADNc ayant la structure 
22
GGGCGCAGCGGTCCTCTCTAT

95
CTAGCTCCAGCCTCTCGCCTGCGCCCACTCCCCGCGTCCCGCTCCTAGCCGACC ATG GCC GGG CCC CTG CGC
Met Ala Gly Pro Leu Arg
-26 -21

155
GCC CCG CTG CTC CTG CTG GCC ATC CTG GCC GTG GCC CTG GCC GTG AGC CCC GCG GCC GGC
Ala Pro Leu Leu Leu Leu Ala Ile Leu Ala Val Ala Leu Ala Val Ser Pro Ala Ala Gly
-1

215
TCC AGT CCC GGC AAG CCG CCG CGC CTG GTG GGA GGC CCC ATG GAC GCC AGC GTG GAG GAG
Ser Ser Pro Gly Lys Pro Pro Arg Leu Val Gly Gly Pro Met Asp Ala Ser Val Glu Glu
1 20

275
GAG GGT GTG CGG CGT GCA CTG GAC TTT GCC GTC GGC GAG TAC AAC AAA GCC AGC AAC GAC
Glu Gly Val Arg Arg Ala Leu Asp Phe Ala Val Gly Glu Tyr Asn Lys Ala Ser Asn Asp
40

335
ATG TAC CAC AGC CGC GCG CTG CAG GTG GTG CGC GCC CGC AAG CAG ATC GTA GCT GGG GTG

60

395
AAC TAC TTC TTG GAC GTG GAG CTG GGC CGA ACC ACG TGT ACC AAG ACC CAG CCC AAC TTG

80

455
GAC AAC TGC CCC TTC CAT GAC CAG CCA CAT CTG AAA AGG AAA GCA TTC TGC TCT TTC CAG

100
 
515
ATC TAC GCT GTC CCT TGG CAG GGC ACA ATG ACC TTG TCC AAA TCC ACC TGT CAG GAC GCC

120

593
TAG GGGTCTGTACCGGGCTGGCCTGTGCCTATCACCTCTTATGCACACCTCCCACCCCCTCTATTCCCACCCCTGGAC

672
TGGTGGCCCCTGCCTTGGGGAAGGTCTCCCCATGTGCCTGCACCAGGAGACAGACAGAGAAGGCAGCAGGCGGCCTTTG

751
TTGCTCAGCAAGGGGCTCTGCCCTCCCTCCTTCCTTCTTGCTTCTCATAGCCCCGGTGTGCGGTGCATACACCCCCACC

777
TCCTGCAATAAAATAGTAGCATCCCC
 ou une fraction de cette structure.

3. Séquence d'ADN caractérisée en ce qu'elle comprend des codons qui codent pour une 3-déshydroxycystatine C modifiée de la façon indiquée dans la revendication 1.

4. Séquence d'ADN caractérisée en ce qu'elle comprend des codons qui codent pour une 3-déshydroxycystatine C avec une ou plusieurs des modifications suivantes:
1. Gly¹¹ → résidu d'aminoacide à charge positive, par exemple Arg
2. Gly¹¹ → résidu d'aminoacide à charge négative, par exemple Glu
3. G1y¹¹ → résidu d'aminoacide fortement hydrophobe, par exemple Trp
4. 4.Gly¹¹ → Ala¹¹, 5Gly¹¹ → Ser¹¹
5. Remplacement de simples aminoacides, par exemple
a) Arg⁸ → Glu
b) Leu⁹ → Arg, Glu
c) Val¹⁰ → Arg, Glu
d) Gly¹ → Arg, Glu
e) Pro¹³ → Arg, Glu, Trp
f)Art ¹⁴ → Arg, Glu
6. Remplacement d'une région entière, par exemple Arg⁸ - Met¹⁴ → Gly-Gly-Gly-Gly-Gly-Gly-Gly
7. Délétion de Arg⁶ - Met¹⁴
8. Cys⁸³ → Gly
9. Cys⁹⁷ → Gly
10. Délétion de Asn⁶¹ - Ala¹⁰
11. Délétion de Asp⁸¹ - Ala¹⁰
12. Délétion de Ile¹⁰¹ - Ala¹⁰

5. Procédé de production de 3-déshydroxycystatine C ou d'un de ses dérivés selon la revendication 1, caractérisé en ce que l'on cultive un organisme hôte contenant une séquence d'ADN selon les revendications 2, 3 ou 4 dans un substrat, et on extrait ensuite à partir de la culture la 3-déshydroxycystatine C ou son dérivé formé.

6. Plasmide à introduire dans un organisme hôte qui est utilisé pour la fermentation de biosynthèse de la 3-déshydroxycystatine C ou d'un de ses dérivés selon la revendication 1, caractérisé en ce qu'il contient la structure d'ADN décrite dans les revendications 2, 3 ou 4, ainsi que des séquences de promoteur, signal, etc.

7. Microorganisme caractérisé en ce qu'il contient un plasmide selon la revendication 6.

8. Préparation thérapeutique, caractérisée en ce qu'elle contient de la 3-déshydroxycystatine C ou un de ses dérivés tels que définis dans la revendication 1.
